Europäisches Patentamt

(19) European Patent Office

Office européen des brevets

(11) Publication number : **0 465 131 A2**

(12) **EUROPEAN PATENT APPLICATION**

(21) Application number : 91305832.7

(22) Date of filing : 27.06.91

(51) Int. Cl.⁵ : **C12N 11/02,** // C02F3/00

(30) Priority : 28.06.90 JP 168186/90

(43) Date of publication of application :
08.01.92 Bulletin 92/02

(84) Designated Contracting States :
**CH DE FR GB IT LI NL**

(71) Applicant : **EARTHNICS CORPORATION**
**9-9 Soto-Kanda 1-chome**
**Chiyoda-ku, Tokyo (JP)**

(72) Inventor : **Kuroda, Masao**
**15-10 Kotobukicho**
**Ashikaga-shi, Tochigi-ken (JP)**
Inventor : **Uchida, Hiroshi**
**3-2074 Honjoh**
**Ashikaga-shi, Tochigi-ken (JP)**
Inventor : **Tanaka, Koji**
**432-9 Masunocho**
**Okayama-shi, Okayama-ken (JP)**
Inventor : **Terada, Tatsuo**
**21-1-707 Aobaoka, Kita**
**Suita-shi, Osaka (JP)**

(74) Representative : **Daley, Michael John et al**
**F.J. CLEVELAND & COMPANY 40/43 Chancery**
**Lane**
**London, WC2A 1JQ (GB)**

(54) **Biocatalyst carrier for bioreactor and method of treatment with biocatalyst.**

(57) A biocatalyst carrier for a bioreactor contains an absorbent material and has the effect of a microfilter. Liquid or gas is treated with the biocatalyst carrier by a method which includes causing a liquid containing a biocatalyst to contact the biocatalyst carrier containing an absorbent material having a degree of swelling of at least 2 and possessing the effect of a microfilter, thereby causing the carrier to absorb the liquid and consequently allowing the biocatalyst in the liquid to be attached to the surface of the carrier, then bringing the carrier into contact with at least one of the liquid and the gas to be treated.

FIG.I(a)

FIG.I(b)

EP 0 465 131 A2

This invention relates to a carrier for a biocatalyst used in a bioreactor operated for the production and recovery of economically valuable substances, and the decomposition of environment-polluting substances and the sewage treatment by the biochemical reactions of such biocatalysts as microorganic cells, and animal and plant tissues, to a carrier having a biocatalyst immobilized thereon and to a method of treatment of gas and/or liquid using the biocatalyst immobilized on the carrier. Water to be treated in the present invention includes a solution, liquid and liquor used in the description.

The method of treatment which comprises immersing a carrier carrying an anaerobic microorganism as a biocatalyst in a liquid held in a treating tank supplied with the liquid containing organic matter thereby effecting decomposition and removal of the organic matter has been known heretofore to the art.

The anaerobic treatment mentioned above requires a preparatory procedure which comprises first placing a liquid containing the anaerobic microorganism in the treating tank, immersing the carrier in the liquid, and allowing the anaerobic microorganism to be attached to the carrier and propagate thereon to the extent of acquiring an activity necessary for the treatment aimed at before the treatment is started.

Heretofore, plastic, ceramic, conglomerate, synthetic fiber, various covering and immobilizing materials, etc. have been used for the carrier. After being immersed in the liquid held in the treating tahk, it is necessary to let the carrier stand for some months before the start of the operation. Besides, a no-load operation has to be continued in the treating tank until the anaerobic microorganism is amply acclimated. The conventional carrier has thus entailed not only a waste of time before the activity of the catalyst reaches the prescribed level required for starting the operation but also a waste of energy during the no-load operation.

This invention aims at eliminating the problem mentioned above by providing a biocatalyst carrier which permits very quick immobilization thereon of even an anaerobic microorganism difficult to immobilize and which allows a dramatic increase in the amount of a biocatalyst that can be attached thereto.

Another object of this invention is to provide a biocatalyst-immobilized carrier (i.e. a carrier having a biocatalyst immobilized thereon) which allows highly efficient contact of the catalyst with a liquid and/or gas under treatment, enables the biocatalyst to maintain its high activity over long periods, and ensures easy handling.

Another object of this invention is to provide a method of treatment by use of the biocatalyst-immobilized carrier mentioned above, which reduces the amount of time and energy wasted in the propagation of the catalyst and which effects the treatment of the liquid and/or gas effectively and economically.

The biocatalyst carrier for a bioreactor contemplated by this invention for realizing the objects set out above contains an absorbent material and manifests the effect of a microfilter. The absorbent material is desired to possess a degree of swelling of at least 2 and absorb water so fast as to require only a few seconds to several minutes until saturation.

The biocatalyst-immobilized carrier is obtained by causing a carrier containing an absorbent material possessing a degree of swelling of at least 2 and manifesting the effect of a microfilter to contact a biocatalyst-containing liquid thereby allowing the carrier to absorb the liquid and consequently causing the biocatalyst in the liquid to be attached fast to the surface of the carrier, and thereafter enclosing and immobilizing the biocatalyst attached fast to the surface of the carrier with an immobilizing material.

The method of treatment by the use of the biocatalyst described above consists in causing the biocatalyst attached to the carrier described above to contact a liquid or gas.

The biocatalyst carrier exhibiting the effect of a microfilter as described above possesses a high degree of swelling and absorbs water at a high rate. It therefore excels in the ability to immobilize a biocatalyst suspended in a slurry. When it is held in contact with a biocatalyst-containing liquid, the liquid is rapidly absorbed by the absorbent material of the carrier and the solid component of the liquid is attached to and accumulated on the surface of the carrier by the microfilter effect.

Since the biocatalyst carrier of this invention is capable of quick effective immobilization of the biocatalyst in a substantially saturated state, it obviates the necessity for propagating the biocatalyst and enables the actual operation to be immediately started by establishing contact between itself and the liquid or gas subjected to the treatment and enables an economical and effective treatment.

The other objects and characteristics of this invention will become apparent from the detailed description to be given hereinbelow with reference to the accompanying drawings.

Figure 1(a) is a perspective view illustrating an example of the biocatalyst carrier according to the present invention.

Figure 1(b) is a perspective view illustrating another example of the biocatalyst carrier according to the present invention.

Figure 2(a) is a graph showing changes in the amount of digestion gas generated by the treatment of liquid using the biocatalyst carrier of the present invention.

Figure 2(b) is a graph showing changes in the amount of an anaerobic microorganism attached to the biocatalyst carrier of the present invention.

Figure 3 is a schematic cross section illustrating a fluidized layer of the biocatalyst-immobilized carrier according to the present invention which is used as a fluidized bed.

Figure 4 is a partially cutaway perspective view illustrating a cylindrical cartridge packed with the biocatalyst-immobilized carrier according to the present invention.

Figure 5 is a perspective view illustrating an example of the biocatalyst carrier according to the present invention in the form of a corrugated sheet.

Figure 6 is a perspective view illustrating another example of the biocatalyst carrier according to the present invention in a spiral form, which is stretched in a frame.

The biocatalyst carrier for a bioreactor contemplated by this invention contains an absorbent material and exhibits the effect of a microfilter.

The "effect of a microfilter" of the biocatalyst carrier refers to a filtration effect originating in local flow of water generated by the high-speed (rate) water-absorbing ability of the absorbent material in the direction normal to the surface of the absorbent material. By "degree of swelling" is meant the ratio of the weight of water absorbed to the weight of the absorbent material.

Since the effect of a microfilter and the ability to immobilize the biocatalyst suspended in the slurry are both heightened in proportion as the degree of swelling increases and the rate of water absorption accelerates, the degree of swelling is required to be at least 2, preferably more than 5 and the period of water absorption up to saturation is desired to be in the range of from a few seconds to several minutes.

The ratio of the amount of the absorbent material contained in the carrier to the amount of this carrier is not critical so long as the biocatalyst carrier is endowed with the effect of a microfilter. The absorbent material may be suitably used in combination with a non-adsorbent material, in consideration of the degree of swelling of the absorbent material.

The forms which the absorbent material can assume include powder, granules, pellets, fluffy mass, fibers, film, and sheet. The form most convenient for the occasion is used. The absorbent material in the form of powder, granules, or pellets may be used as a filler for a fluidized bed. The absorbent material in the form of powder, granules, pellets, or fluffy mass may be retained as contained in a meshed pouch or tube or retained as nipped between two opposed sheets of non-woven fabric in consideration of the convenience of handling during the insertion into or removal from a treating tank. When the absorbent material is in the form of filaments or long-staple fibers, it may be put to use directly in its unmodified form. When necessary, the filaments or fibers may be fabricated into linear materials such as yarns, cords, ropes, or rods or into sheetlike materials such as paper, non-woven fabric, or knit or woven fabric. They may be otherwise fabricated into spirally braided cords, for example.

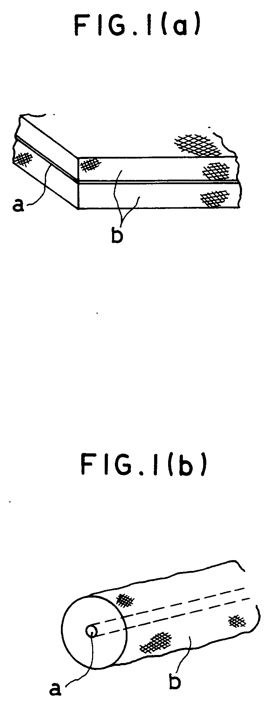

Particularly from such points as ease of handling, fabricability in desired size and shape, ability to carry a biocatalyst, and efficiency of contact with the liquid under treatment it is desirable to use an absorbent material in the form of fibers, preferably fibers arranged in two layers, one being an absorbent layer and the other a non-absorbent layer, and fabricated in the form of sheet or spirally braided cord. For example, a sheet or film of non-absorbent material $\underline{a}$ is sandwiched by absorbent materials $\underline{b}$ as shown in Figure 1(a) to form a carrier of sandwiched structure. Another example for constituting a carrier is as shown in Figure 1(b) in which a wirelike non-absorbent material $\underline{a}$ is clad with an absorbent fiber material $\underline{b}$. By using the non-absorbent material is used as a core, as described above, the carrier is improved in strength and is easy to handle even when the absorbent material having a biocatalyst carried thereon is swollen. For the purpose of enhancing the ability to carry a biocatalyst, the absorbent material is desired to be given an irregular surface. For this purpose, it suffices to form minute hills and valleys as by subjecting powder, granules, or fibers to a plasma treatment or extrude fibers in such a cross-sectional shape as the letter Y, a cross, or a pentacle by selection of the nozzle orifice.

The raw material of the absorbent material may be freely selected from among various substances including both natural substances and synthetic substances so long as the produced absorbent material possesses the required swelling property. The raw materials which are usable advantageously herein include starch-acrylate graft polymers, copolymers of acrylonitrile with (meth)acrylates, copolymers of such dibasic acids as maleic acid, non-absorbent materials coated with such hydrophilic polymers as sodium polyacrylate, and acrylic fibers and cellulose fibers modified for the acquisition of absorbency, for example.

Though the kind of a biocatalyst attached to the carrier is not critical, the biocatalyst is desired from the practical point of view to be an anaerobic microorganism which has heretofore required a long time for immobilization.

Materials which are effectively usable for covering and immobilizing a biocatalyst attached to the surface of the carrier include cellulose, dextran, starch, agar, carrageenan, alginic acid and derivatives thereof, gelatin, albumin, collagen, tannin, PVA, photo-cross-linking resins, urethane, polyacryl amide gel, polyethylene glycol, and epoxy resins, for example. The biocatalyst attached to the carrier is immobilized on the carrier by applying, spraying or showering the immobilizing material onto the biocatalyst or by immersing the carrier in the immobilizing material.

Three types of non-woven fabric of a two-layer construction (absorbent outer layer and non-absorbent inner layer) incorporating therein a prescribed amount of ultraabsorbent fibers (produced by Nippon-Exlan Kogyo K.K. in Japan and marketed as "LANSEAL") and consequently exhibiting varying degrees of swelling of 7, 20 and 150 were compared with other materials both as carriers with respect to the capacity for attachment of an anaerobic microorganism by a procedure which consisted of immersing a sample carrier in a slurry containing 3,840 mg of the anaerobic microorganism (methane-fermenting bacteria) per liter for three minutes, removing the carrier from the slurry, cleaning the wet carrier with ultrasonic waves, and measuring the concentration of the anaerobic microorganism contained in the washings in terms of TOC. The results are shown in Table below.

Table 1

|  | Degree of swelling | Amount of attached microorganism $(mg/cm^2)$ |
|---|---|---|
| LANSEAL-containing non-woven fabric | 7 | 0.42 |
| Do. | 20 | 0.50 |
| Do. | 150 | 1.47 |
| Paperlike non-woven fabric of carbon fibers | 0.1 | 0.03 |
| Non-woven fabric of polyester | 0.79 | 0.06 |
| Absorbent polyurethane | 31 | 0.25 |

For ascertaining the effect of the concentration of the anaerobic microorganism in the slurry upon the amount of attachment of the anaerobic microorganism, four sheets of the aforementioned LANSEAL-containing non-woven fabric having a degree of swelling of 150 were prepared, respectively immersed for three minutes in a liquid of a slurry concentration of 1,360 mg/liter, solutions obtained by diluting this liquid to two and three times the original volume with distilled water, and a liquid of a slurry concentration of 3,840 mg/liter, removed from the liquids, and thereafter processed in the same manner as described above to determine the concentration of the anaerobic microorganism in the washings in terms of TOC. The results were as shown in Table 2 below.

## Table 2

| Slurry concentration (mg/liter) | Amount of attached microorganism (mg/cm$^2$) |
|---|---|
| $\dfrac{1360}{3} \fallingdotseq 450$ | 0.68 |
| $\dfrac{1360}{2} = 680$ | 0.85 |
| 1360 | 1.2 |
| 3840 | 1.47 |

These test results indicate that the degree of swelling ($\alpha$) can be defined by the following equation using the amount of liquid absorbed per unit surface area of the material

$$\alpha = \frac{W}{\omega} \quad (1)$$

where W stands for the amount of the liquid absorbed (g/cm$^2$) and $\omega$ for the weight of the material (g/cm$^2$).

In a absorbent material having a degree of swelling exceeding 2, since the liquid is rapidly absorbed by the material, it is believed that the liquid produces a local flow on the surface of the material in the direction perpendicular to the surface and that the amount of the liquid, namely the flux (J), flowing per unit surface area in the direction perpendicular to the surface is directly proportional to the n'th power of the degree of swelling, as expressed in Equation 2

$$J \, \alpha \, (\alpha s - \alpha)^n \quad (2)$$

(where s stands for saturation).

When the liquid is in the form of slurry, the solids in the slurry are conveyed as entrained by the local flow of liquid, so that, while the liquid is being absorbed by the material, the solids are retained on the surface of the material by virtue of the microfilter effect and are eventually attached fast thereto. If the slurry has a high solids content, the solids pile up in the form of a cake on the surface of the material.

The rate of attachment (F) of the solids to the surface of the material is believed to be directly proportional-.either to the amount of movement of the liquid by the microfilter effect or the amount of absorption and the slurry concentration as expressed in Equation 3

$$F \, \alpha \, J \cdot C^m = C^m (\alpha s - \alpha)^n \quad (3)$$

(where C stands for the slurry concentration).

The total amount of attachment of the solids (M) may be expressed by approximation as shown in Equation 4

$$M = \beta \cdot C^m \cdot \alpha s^n \quad (4)$$

(where $\beta$ is a coefficient).

An example will now be described in which a LANSEAL-containing non-woven fabric having a degree of swelling of 150 was used for the attachment of an anaerobic microorganism.

The non-woven fabric mentioned above was cut into strips a few cm to a few m wide and some tens of cm to several m long and, in a state exhibiting the water absorbing property, immersed in a tank used for culturing an anaerobic microorganism. The reason for immersing the strips in the liquid in the dry absorbent state is that the effect of a microfilter is then manifested fully. If the strips are caused to absorb water to the state of saturation in advance of the immersion in the liquid, then the initial amount of attachment of the anaerobic microorganism is decreased. The concentration of the anaerobic microorganism in the liquid with which the biocatalyst carrier is brought into contact is desired to be about 100 mg/liter.

After a few minutes' immersion of the strips of non-woven fabric in the liquid, the microorganism was attached fast to the surface of the strips. By "fast attachment of the anaerobic microorganism" is meant that the anaerobic microorganism accumulates in the form of a layer and attaches thereto so as to cover the surface of the fibers.

During the immersion of the strips of LANSEAL-containing non-woven fabric having a degree of swelling of 150 in the liquid of an anaerobic microorganism (methane-fermenting bacteria) slurry concentration of 1,360 mg/liter, the effect of the immersion time on the amount of attachment of the anaerobic microorganism was examined. The results were as shown in Table 3. The amounts of attachment reported in the table represent the relative value of the amount of the microorganism attached during three minutes' immersion, determined by washing the immersed strips with ultrasonic waves and measuring the TOC concentration of the microorganism suspended in the washings.

Table 3

| Immersion time | 3 minutes | 10 minutes | 30 minutes | 60 minutes |
|---|---|---|---|---|
| Relative amount of attachment | 1 | 0.8611 | 0.8055 | 0.6041 |

It will be noted from the results shown in Table 3 that the amount of attachment peaked at an immersion time of three minutes and decreased as the immersion time increased above three minutes. It is supposed that the explanation for this is that since the degree of swelling of the non-woven fabric was 150, the microfilter effect was manifested strongly during the first three minutes of immersion so that the anaerobic microorganism was attached substantially to maximum saturation, but that as the immersion time was protracted past the three minutes, the microfilter effect diminished and disappeared so that part of the anaerobic microorganism once attached to the strips of non-woven fabric was liberated from the fabric and passed back into the liquid.

Therefore, the contact time with the carried biocatalyst has to be suitably selected in accordance with the degree of swelling of the absorbent material as a component of the carrier. The results produced by this contact does not necessarily get better with increasing contact time.

After the amount of the anaerobic microorganism attached to the surface of the strips reached a prescribed level, the strips were set in place inside the tank being used for anaerobic treatment of a given liquid. The actual treatment operation was started by initiating supply of the liquid to the tank and acclimating the microorganism. In the case where a gas is to be treated, the strips are stretched inside a frame or packed in a cylindrical cartridge so that they are effectively in contact with the gas.

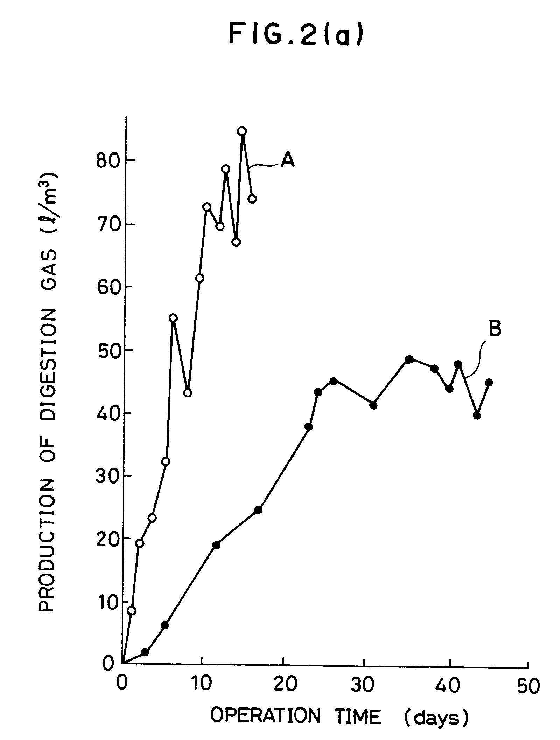

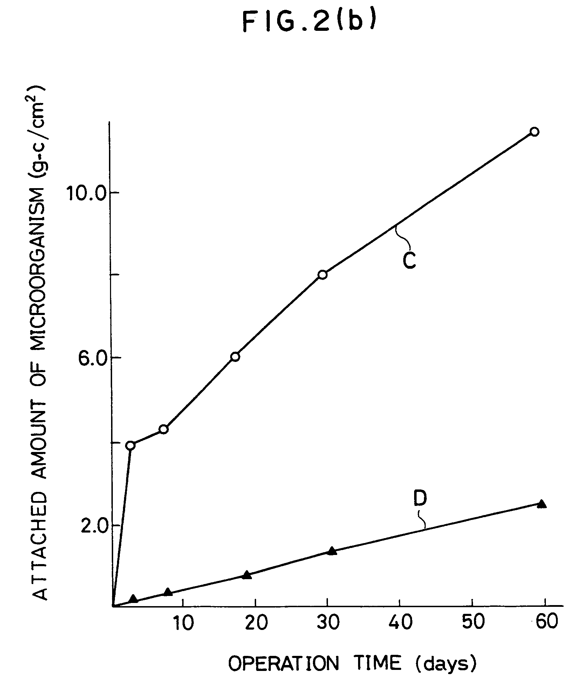

Figure 2(a) is a graph showing time-course changes in the amount of digestion gas generated in an experiment conducted by subjecting a LANSEAL-containing non-woven fabric having a degree of swelling of 150 to a three-minute immersion in a liquid of an anaerobic microorganism (methane-fermenting bacteria) slurry concentration of 240 mg/liter, removing the fabric from the liquid, setting the fabric in a treating tank to which a water having BOD in the range of 130 to 180 mg/liter (TOC 260 to 360 mg/liter) was supplied, and starting the operation of this treatment.

Since the anaerobic microorganism was attached substantially to saturation on the LANSEAL-containing non-woven fabric, the generation of the digestion gas began immediately after the start of the operation as indicated by the curve $\underline{A}$. The generation of the digestion gas passed the mark of more than 70 liters/$m^3$ of the liquid after about ten days' operation and leveled off thereafter.

For comparison, a bulky non-absorbent non-woven fabric was adopted as a carrier, immersed for ten minutes in a liquid of an anaerobic microorganism slurry concentration of 10,000 mg/liter held in a tank, removed from the liquid, set in place in a treating tank, and used for treatment therein. The amount of digestion gas generated during this treatment was measured. The results were as shown by the curve $\underline{B}$ in Figure 2(a). In spite of the use of a carrier which had been immersed in a liquid having an anaerobic microorganism slurry concentration more than 40 times that of the carrier of this invention for a period more than three times that used for the carrier of the present invention, generation of the digestion gas was observed only after the elapse of three days following the start of the operation and the amount of generation of this gas was very small, specifically less than 5 liters/$m^3$ of the liquid or less. About 30 days following the start of the operation, the amount of the generated gas reached a level in the range of from 40 to 50 liters/$m^3$ and leveled off thereafter. The amount of generation was far lower than that in the case of the carrier of the present invention.

After the start of the operation, part of the carrier held in the treating tank was occasionally lifted from the liquid and examined to ascertain the amount of the anaerobic microorganism (methane-fermenting bacteria) attached to the carrier. The results were as shown in Figure 2(b). As indicated by the curve $\underline{C}$ in the graph, the amount of the microorganism attached was about 4.0 mg-C (amount of available carbon)/$cm^2$ on the third day, began to increase sharply on the seventh day, and surpassed 10.0 mg-C/$cm^2$ after the elapse of 60 days.

For comparison, a porous carbon plate having a degree of swelling of 1 was set in a treating tank to which the same liquid as mentioned above was supplied, and used for treatment therein. During the treatment, part of the carbon plate was occasionally lifted from the liquid and examined to ascertain the amount of the anaerobic microorganism attached thereto. The results were as indicated by the curve $\underline{D}$ in Figure 2(b). The amount of the microorganism attached to the carbon plate was virtually nil even after the elapse of three days and only about 2.0 mg-C/cm$^2$ after the elapse of 60 days.

The test results given above clearly show that by optimally controlling the contact time of the anaerobic microorganism with the carrier in accordance with the degree of swelling of the absorbent material, the attached anaerobic microorganism can be acclimated and copiously propagated even immediately after the start of the operation and, owing to the abundance of the microorganism, stable operation of the treatment can be started after a very short time of about 10 days.

In the example cited above, the operation of the treatment was not started until the carrier was immersed in a liquid containing an anaerobic microorganism to cause attachment of the microorganism thereto and the carrier removed from the liquid was set in place in the treating tank. Optionally, the attachment of an anaerobic microorganism to a carrier may be effected by spraying or showering a liquid containing the microorganism onto the carrier or by setting the carrier in the treating tank and allowing this carrier to contact the microorganism-containing liquid by suitable means. The operation of the treatment may be started by initiating the supply of the liquid or gas to be treated to the treating tank in which the carrier coated with the attached layer of the microorganism is set in place. Where an aerobic microorganism is used, it is immobilized on a carrier under aeration.

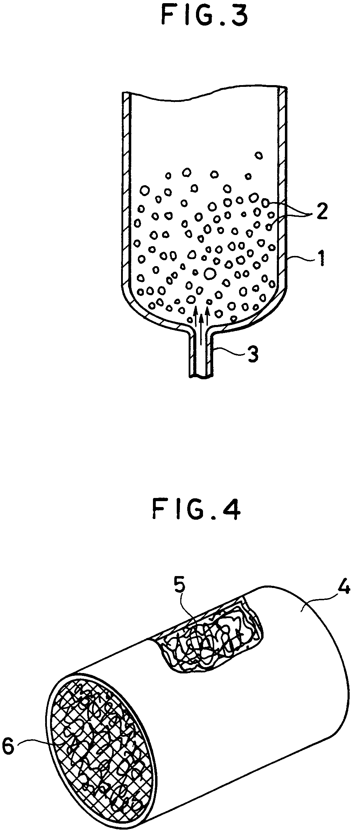

As shown in Figure 3, when the carrier 2 is in the form of powder or granules, it may be placed in a treating column 1 to which the liquid to be treated is supplied upward from a supply inlet 3 and may be immobilized by the upward flow of the liquid to form a fluidized bed. In this case, the conventional carrier of this form has required some months' time for the individual particles to be completed by being independently wrapped with an immobilizing agent or by causing a granule-forming bacteria to be acclimated and attached fast to the individual particles. The carrier of this invention in the form of powder or granules is such that the effect of a microfilter due to the degree of swelling of the carrier with water enables all of the powder or granules to adsorb the biocatalyst quickly (within a matter of a few seconds to several minutes) and immobilize it in a concentrated state.

When the method of covering immobilization is additionally performed, the maximum amount of attachment can be maintained and the efficiency of the attachment can be further enhanced by setting the contact time of the microorganism so as to maximize the amount of attachment due to the microfilter effect and then subjecting the biocatalyst to the covering immobilization with an immobilizing agent.

In the simultaneous adoption of the method of covering immobilization, unlike the conventional method of covering immobilization, since the biocatalyst is already attached fast, it suffices only to cover the attached biocatalyst with a covering immobilizing material to be used in the least possible amount necessary for the sake of surface strength. Thus, this method is capable of improving the efficiency of contact as compared with the conventional method of covering immobilization.

The biocatalyst carrier of this invention contains an absorbent material having a degree of swelling of at least 2 and, owing to the microfilter effect produced by this degree of swelling, enables even the least readily immobilizable anaerobic microorganism, which may require some months for immobilization with an ordinary material, to be immobilized in a very short span of time. It allows a dramatic increase in the amount of the biocatalyst that can be attached and, owing to the consequent increase in the biomass, permits size-reduction of the equipment used for the treatment.

The biocatalyst-immobilized carrier resulting from the covering immobilization of a biocatalyst on a carrier with an immobilizing material not only enhances the efficiency of contact and facilitates the maintenance of high activity of the biocatalyst but also retains the dry state of the enzyme, enables protracted conservation of the biocatalyst and, at the same time, improves the ease of handling.

The activity of the biocatalyst may be improved by having a nutritive substance or an activity-retaining agent incorporated in the form of microcapsules in the immobilizing material.

The method for treating gas or liquid with the biocatalyst carrier according to the present invention will now be described with reference to Figure 4.

A cylindrical cartridge 4 open at the opposite ends and permitting passage of gas or liquid therethrough is packed with a biocatalyst 5 containing an absorbent material. The open ends of the cartridge 4 are stopped up by lids 6 such as a porous plate or a net permitting passage of gas or liquid therethrough. The biocatalyst carrier 5 packed in the cartridge 4 may be in the form of powder, granules, pellets, fluffy mass, fiber, ropes or braided cords. In the case of the biocatalyst carriers 5 in the form of ropes or braided cords, they are inserted into the cartridge 4 in the form of a bundle extending in the lengthwise direction of the cartridge 4. The biocatalyst carrier 5 may be in the form of a planar net or porous film or sheet. In this case, the carrier 5 is punched out into a plurality of disks having a diameter substantially the same as the inside diameter of the cartridge 4, the disks are piled up into a laminate, and the laminate is accommodated into the cartridge.

The cartridge 4 containing the biocatalyst carrier 5 therein is immersed in a culture solution or a culture solution is sprayed or showered into the cartridge 4 for the purpose of allowing the absorbent material to absorb the solution, thereby immobilizing the biocatalyst on the surface of the carrier. The surface of the carrier may be coated with an immobilizing material, when necessary, to cover and immobilize the biocatalyst attached to the surface of the carrier. Muddy water or rank gas to be treated is supplied into the cartridge 4 from one end thereof and, as a result, purified water or deodorized gas having the rank order components decomposed can be obtained at the other end of the cartridge. When rank water is passed through the biocatalyst in the cartridge 4, rank odor components emitted from and dissolved in the rank water can be decomposed at the same time to obtain purified water. In the case where rank gas alone is treated, water is supplemented into the cartridge 4 during the operation so as to prevent the carrier from being dried and an active agent or activity-retaining agent is supplemented to prevent the carrier from lowering its activity.

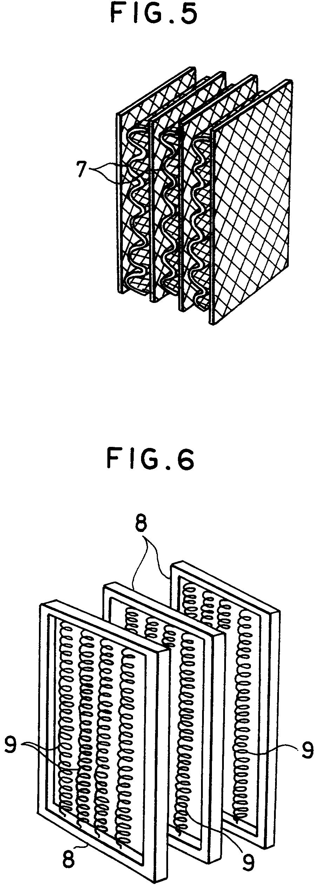

The biocatalyst carrier may be in the form of a corrugated sheet or plate 7 as shown in Figure 5 or of ropes, straps or braided cords strung inside a frame 8 as shown in Figure 6. In either case, a plurality of sheets 7 or frames 8 are piled up into a laminate having a prescribed length for being mounted in treating equipment.

By the treating method according to the present invention, the attachment of a biocatalyst on a carrier is attained very quickly and the actual treatment operation can be started without waste of time or energy otherwise required for a no-load operation. In addition, the biocatalyst carrier ensures easy handling by accommodating it in a container permitting passage of liquid or gas therethrough. Furthermore, by placing the carrier in a treating tank or tower for treating liquid or gas therein and causing the carrier to absorb a culture solution, it is possible to eliminate time and labor consumption required for transporting into the treating tank or tower the carrier which has become heavy in consequence of absorbing the culture solution at a different place and to treat the liquid or gas immediately after the carrier has absorbed the culture solution in the treating tank or tower.

## Claims

1.  A biocatalyst carrier for a bioreactor, characterised in that said carrier contains an absorbent material and possesses the effect of a microfilter.

2.  A biocatalyst carrier according to claim 1, wherein said absorbent material possesses a degree of swelling of not less than 2 and a speed of water absorption enabling the absorption to reach saturation in a few seconds to several minutes.

3.  A biocatalyst carrier according to claim 1, wherein said absorbent material is made of fibers.

4.  A biocatalyst carrier according to claim 1, which comprises a two-layer construction composed of an absorbent material and a non-absorbent material.

5.  A biocatalyst carrier according to claim 1, wherein said absorbent material possesses an irregular surface.

6.  A method for the treatment of liquid or gas by contact with a carrier having a biocatalyst carried thereon, characterised in that a biocatalyst carrier containing an absorbent material having a degree of swelling of at least 2 and possessing the effect of a microfilter is used and that said carrier is brought into contact with a liquid containing a biocatalyst to attach said biocatalyst to the surface of said carrier.

7.  A method according to claim 6, wherein said biocatalyst is an anaerobic microorganism.

8.  A method according to claim 6, wherein said immobilization of said biocatalyst on the surface of said carrier is effected by the immersion of said carrier in said biocatalyst-containing liquid.

9.  A method according to claim 6, wherein said immobilization of said biocatalyst on the surface of said carrier is effected by spraying or showering the biocatalyst-containing liquid onto the surface of said carrier.

10. A method according to claim 6, which further comprises covering and immobilizing said biocatalyst attached fast to the surface of said carrier with an immobilizing material.

11. A biocatalyst-immobilized carrier for a bioreactor, characterised in that a carrier contains an absorbent material having a degree of swelling of at least 2 and possesses the effect of a microfilter, and that a biocatalyst is attached fast to the surface of said carrier and covered and immobilized with an immobilizing material.

12. A carrier according to claim 11, wherein said covering and immobilizing material is at least one member selected from the group consisting of cellulose, dextran, starch, agar, carrageenan, alginic acid and derivatives thereof, gelatin, albumin, collagen, tannin, PVA, optical-cross-linking resins, urethane, polyacryl amide gel, polyethylene glycol, and epoxy resins.

# FIG.1(a)

# FIG.1(b)

# FIG.2(a)

# FIG.2(b)

FIG.3

FIG.4

# FIG.5

# FIG.6